# EUROPÄISCHE PATENTSCHRIFT

(11) **EP 0 411 500 B1**
(45) Veröffentlichungstag und Bekanntmachung des Hinweises auf die Patenterteilung: **22.12.1993**
(21) Anmeldenummer: 90114472.5
(22) Anmeldetag: 27.07.1990
(51) Int. Cl.: B01F 3/02

(54) **Vorrichtung zum Herstellen von Gas/Dampf-Gemischen**
Devive for making gas vapour mixtures
Dispositif pour la fabrication de mélanges gaz-vapeur

(30) Priorität: 02.08.1989 DE 3925580
(43) Veröffentlichungstag der Anmeldung: 06.02.1991
(73) Patentinhaber: HOECHST AKTIENGESELLSCHAFT, 65926 Frankfurt am Main (DE)
(72) Erfinder: Melzer, Werner, Dr., D-6237 Liederbach (DE); Freudenberger, Adalbert, Dr., D-6234 Hattersheim am Main (DE); Strahlendorf, Hans, D-6239 Eppstein/Taunus (DE)

(56) Entgegenhaltungen:
- EP-A- 0 160 314
- DE-A- 3 301 675
- DE-C- 899 346
- FR-A- 1 031 462
- GB-A- 2 029 717

## Beschreibung

Gegenstand der Erfindung ist eine Vorrichtung zum Herstellen von Gas/DampfGemischen mit definierter Zusammensetzung aus mehreren Komponenten in einem Verdampfungsreaktor mit Zuführungen für Trägergas und Flüssigkeit sowie mit einer Leitung zum Abführen des Gas/Dampf-Gemisches.

Gasmeßeinrichtungen sind zu kalibrieren. Hierfür gibt es sogenannte Kalibriergase. Die Kalibrierung mit solchen Gasen ist insofern unbefriedigend, daß bei Messungen von Schadstoffen in der Atmosphäre oder in Abgasen von Verbrennungsanlagen der dort vorhandene Wasserdampfanteil unberücksichtigt bleibt, was zu Fehlmessungen führt bzw. umständliche Berechnungen notwendig macht. Es ist also wünschenswert, ein Eichgas zu verwenden, das einen den Realitäten angepaßten Wasserdampfanteil aufweist.

Nach der DE-C 899 346 ist ein Verfahren zum kontinuierlichen Herstellen eines Gasdampfgemisches von erhöhter Temperatur bekannt, bei dem das Gas in mehreren Stufen fortschreitend höher erhitzt wird und hinter den einzelnen Stufen mit je einer Teilmenge der Gesamtflüssigkeit vermischt wird. Die Teilmenge ist so gewählt, daß sie bis zur nächstfolgenden Temperaturstufe verdampft. Dieses Kaskadenverfahren ist technisch aufwendig. Hier will die Erfindung Abhilfe Schaffen.

Die Erfindung löst die Aufgabe durch die eingangs erwähnte Vorrichtung, die dadurch gekennzeichnet ist, daß der Verdampfungsreaktor aus einem mit einem Hohlraum versehenen Heizblock besteht, der in einem Gehäuse angeordnet ist, sich im Hohlraum eine Rohrwendel für das Trägergas befindet, die wärmeleitend mit dem Heizblock verbunden ist und auf dem Außenmantel des Heizblocks eine zweite Rohrwendel angeordnet ist, in die die Flüssigkeitszuführung und die Rohrwendel für das Trägergas mündet. Die Rohrwendel auf der Außenseite des Heizblocks kann mit einem Innenkörper (z.B. aus PTFE) versehen sein, um die Strömung an der Rohrwand zu halten. Die Leitung zum Abführen des Gas/Dampf-Gemisches kann mit einem Heizmantel versehen sein. Die Gaszuführung kann einen Druckregler, eine Trockeneinrichtung und einen Mengenregler aufweisen, und die Flüssigkeitszuführung mit einer Dosiereinrichtung versehen sein.

Die in dieser Vorrichtung hergestellten Gas/Dampf-Gemische eignen sich beispielsweise zum Kalibrieren von Einrichtungen zum Messen der HCI-Emission auf infrarotoptischer Basis, wie sie in Schlamm- bzw. Müllverbrennungsanlagen verwendet werden oder von Feuchtemeßanlagen, oder von Meßanlagen zur Bestimmung von Alkohol in Luft.

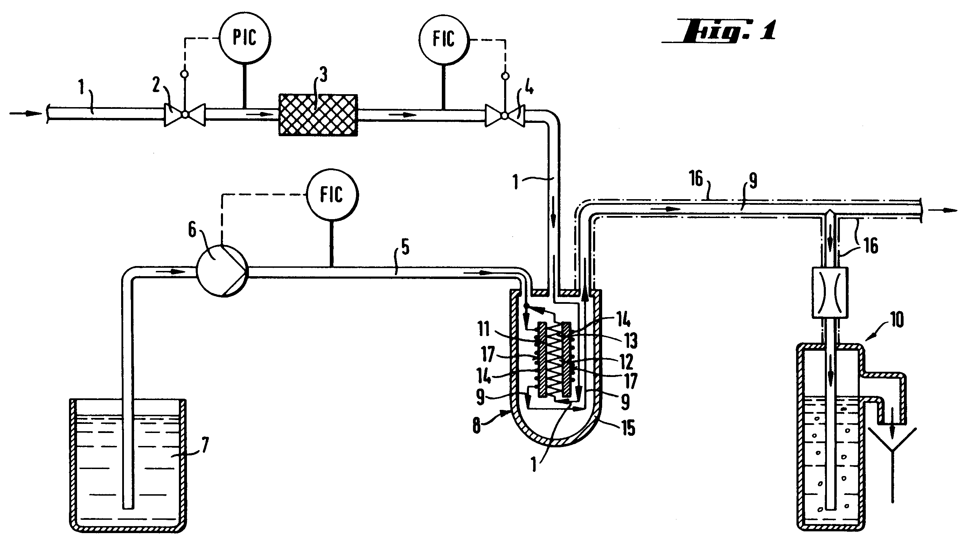

Im folgenden wird die Erfindung anhand von Zeichnungen und Diagrammen näher erläutert. Es zeigt
- Figur 1: ein Funktionschema der erfindungsgemäßen Vorrichtung,

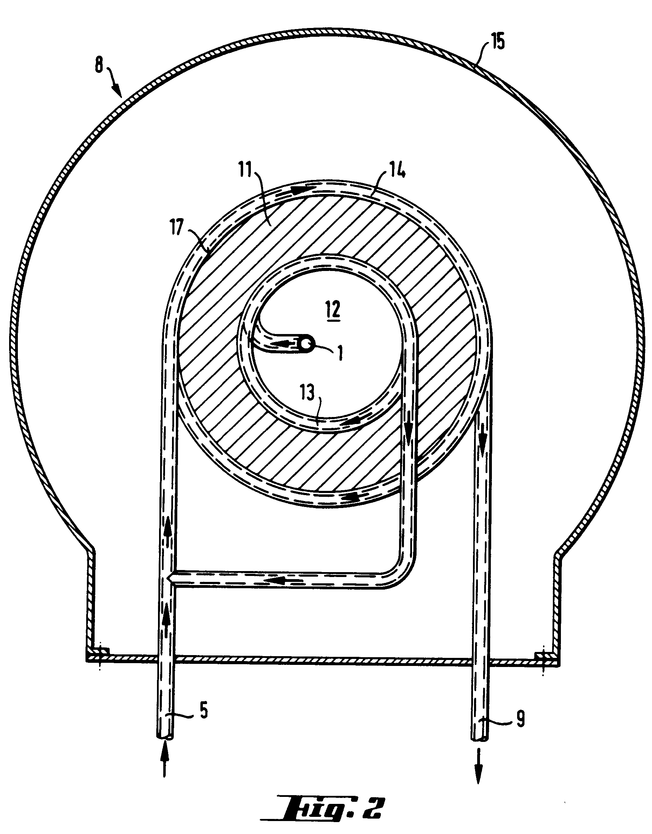

- Figur 2: den Verdampfungsreaktor ohne stirnseitige Gehäuseplatte,
- Figur 3: die Mischvorschrift für eine Flüssigkeitsvorlage bestehend aus Wasser und HCl - Spuren für verschiedene Wasserdampfgehalte in der Luft und verschiedene Temperaturen,
- Figur 4: die Fördermengen Trägergas (Luft) und Flüssigkeitsvorlage gemäß Figur 3 in Abhängigkeit vom Wasserdampfgehalt im Meßgas für einen bestimmten HCI-Gehalt,
- Figur 5: die Mischvorschrift für eine Flüssigkeitsvorlage bestehend aus Methanol und Wasser bei verschiedenen Wasserdampfgehalten in der Luft und einer Temperatur von 150°C und
- Figur 6: die Fördermengen Trägergas (Luft) und Flüssigkeitsvorlage gemäß Figur 5 in Abhängigkeit vom Wasserdampfgehalt im Meßgas für verschiedene Methanolgehalte.

Das Gas/Dampf-Gemisch mit definierter Zusammensetzung seiner Komponenten wird in einem Verdampfungsreaktor 8 hergestellt. Dieser Verdampfungsreaktor ist mit Zuführungen 1, 5 für Trägergas und Flüssigkeit und einer Leitung 9 zum Abführen des Gas/Dampf-Gemisches (Eichgas) versehen. In der Zuführung 1 ist eine Druckregelung 2, eine Gastrockeneinrichtung 3 sowie eine Gasmengenregelung 4 vorgesehen, in Zuführung 5 eine Einrichtung 6 für konstante Mengendosierung der Flüssigkeit. Im Verdampfungsreaktor 8 wird das Gas in einer im Hohlraum 12 des Heizblocks 11 wärmeleitend mit dem Heizblock 11 verbundenen Rohrwendel 13 vorgewärmt und anschließend mit der Flüssigkeit aus der Flüssigkeitsvorlage 7 vermischt und in einer zweiten Rohrwendel 14, die auf dem Außenmantel 17 des Heizblocks 11 wärmeleitend angeordnet ist, verdampft. In der Dampfleitung 9 kann eine Überströmeinheit 10 angeordnet sein, mit der das Gas/Dampf-Gemisch (Eichgas) dem Verbraucher in der jeweils benötigten Menge zugeführt werden kann. Der Heizblock 11 ist in einem mit Isolationsmaterial gefüllten (nicht dargestellt) Gehäuse 15 angeordnet. Leitung 9 und/oder Überströmeinheit 10 können mit einem Heizmantel 16 versehen sein.

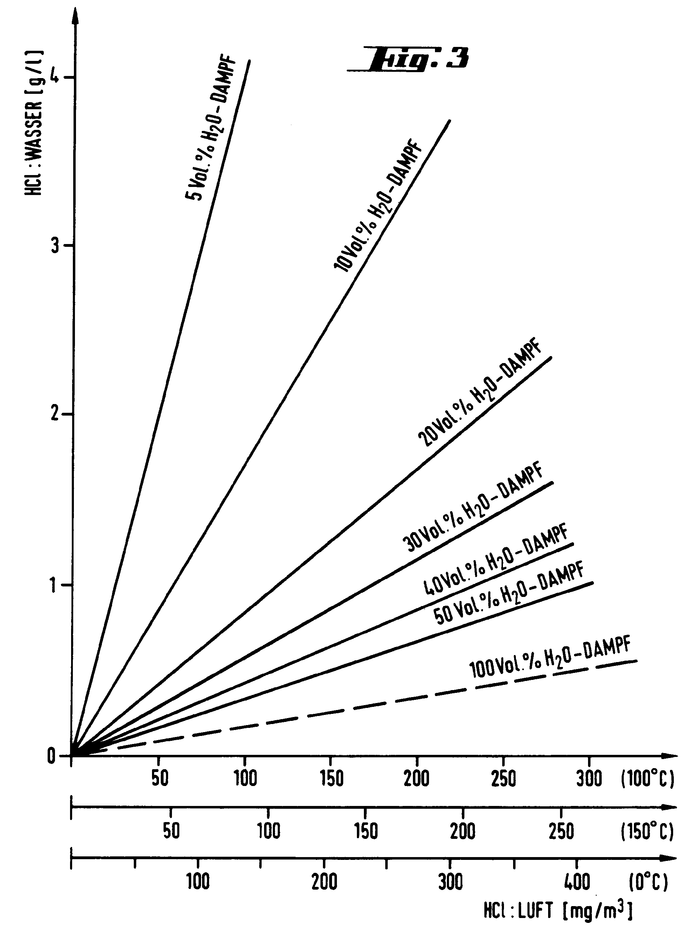

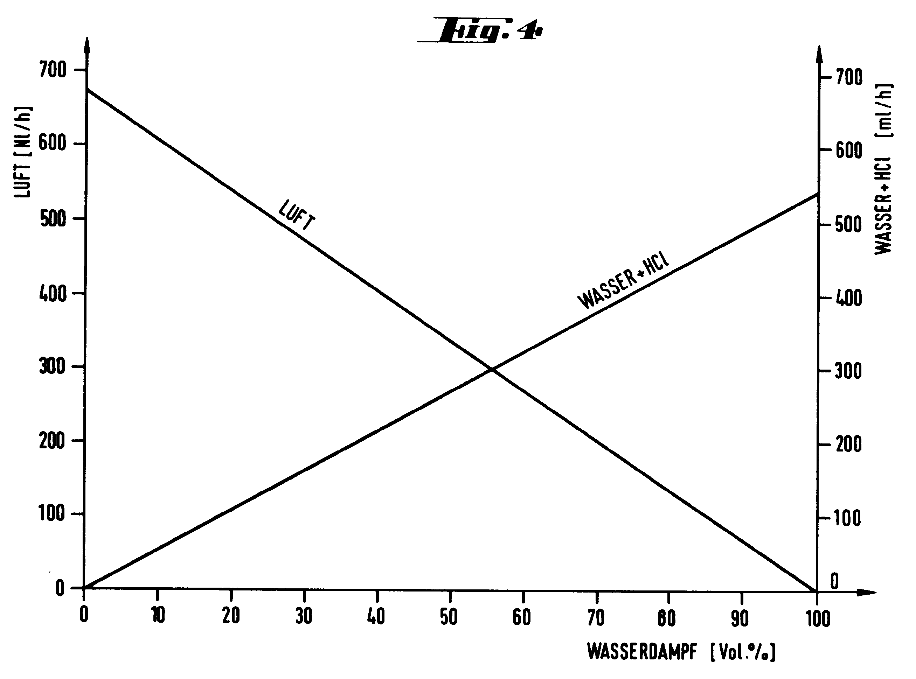

Die Zusammensetzung der Flüssigkeitsvorlage und die Wahl der gasförmigen und flüssigen Mengenströme wird durch Mischvorschriften festgelegt. So zeigt Figur 3 die Mischvorschrift für eine Flüssigkeitsvorlage bestehend aus Wasser und HCl Spuren im Bereich von 0 bis 400 mg HCl pro m³ Luft bei gleichzeitiger Anwesenheit von Wasserdampf in der Luft. Soll das Eichgas beispielsweise 140 mg HCl pro m³ Luft mit einem Wasserdampfgehalt von 30 Vol.-% bei 100°C aufweisen, so muß gemäß Figur 3 die Flüssigkeitsvorlage 0,8 g HCl pro Liter Wasser enthalten. Nach Figur 4 ist dem Verdampfungsreaktor 8 über Zuführung 1 ein Trägergasstrom (Luftstrom) von 470 Nl/h und über Zuführung 5 ein Flüssigkeitsstrom von 165 ml/h zuzuführen.

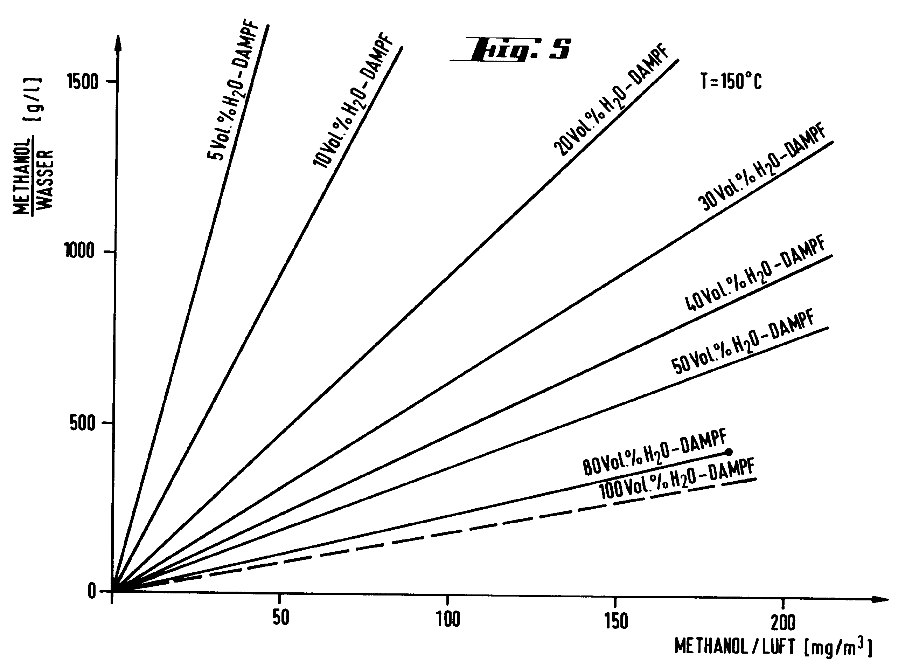

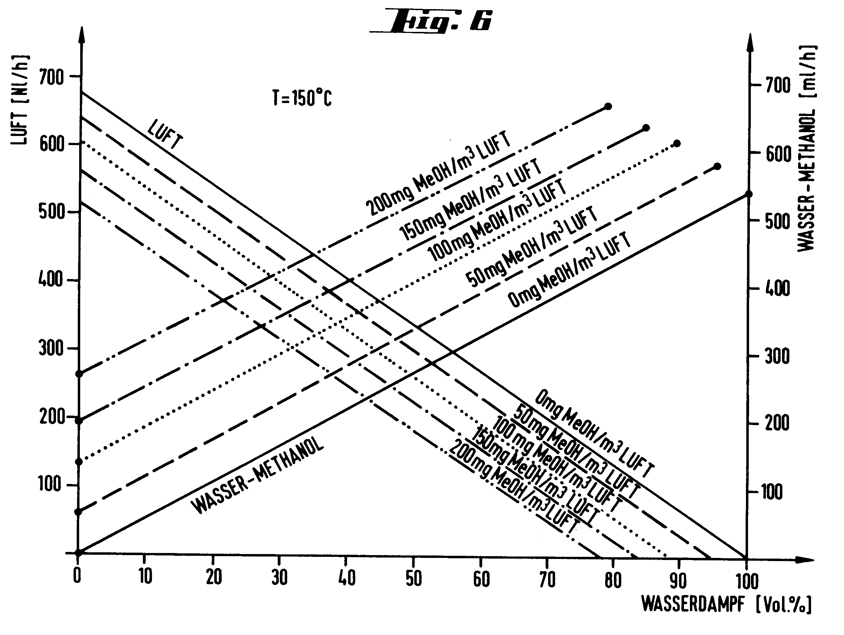

Figur 5 zeigt die Mischvorschrift für eine Flüssigkeitsvorlage bestehend aus Wasser und Methanol für verschiedene Wasserdampfgehalte in der Luft bei 150°C. Soll das Gas/Dampf-Gemisch (Eichgas) bei 150°C beispielsweise 150 mg Methanol pro m³ Luft mit einem Wasserdampfgehalt von 40 Vol.-% aufweisen, so muß die Flüssigkeitsvorlage gemäß Figur 5 720 g Methanol pro Liter Wasser enthalten. Nach Figur 6 ist dem Verdampfungsreaktor 8 über Zuführung 1 ein Trägergasstrom (Luftstrom) von 295 Nl/h und über Zuführung 5 ein Flüssigkeitsstrom von 405 ml/h zuzuführen.

## Patentansprüche

1. Vorrichtung zum Herstellen von Gas/Dampf-Gemischen mit definierter Zusammensetzung aus mehreren Komponenten in einen Verdampfungsreaktor (8) mit Zuführungen (1, 5) für Trägergas und Flüssigkeit sowie mit einer Leitung (9) zum Abführen des Gas/Dampf-Gemisches, dadurch gekennzeichnet, daß der Verdampfungsreaktor (8) aus einem mit einem Hohlraum (12) versehenen Heizblock (11) besteht, der in einem Gehäuse (15) angeordnet ist, sich im Hohlraum (12) eine Rohrwendel (13) für das Trägergas befindet, die wärmeleitend mit dem Heizblock (11) verbunden ist und auf dem Außenmantel (17) des Heizblockes (11) eine zweite Rohrwendel (14) wärmeleitend angeordnet ist, in die die Flüssigkeitszuführung (5) und die Rohrspirale (13) für das Trägergas mündet.

## Claims

1. An apparatus for the preparation of gas/vapor mixtures of defined composition from a plurality of components in an evaporation reactor (8) with feeds (1, 5) for carrier gas and liquid and also a line (9) for withdrawing the gas/vapor mixture, wherein the evaporation reactor (8) comprises a heating block (11) provided with a cavity (12), which block is arranged in a housing (15), and in the cavity (12) is located a helical tube (13) for the carrier gas, which is connected heat-conductively with the heating block (11), and on the outer surface (17) of the heating block (11) is arranged heat-conductively a second helical tube (14) into which the liquid feed (5) and the helical tube (13) for the carrier gas discharge.

## Revendications

1. Dispositif pour préparer des mélanges gaz/vapeur possédant une composition définie à partir de plusieurs constituants dans un réacteur d'évaporation (8) comportant des systèmes d'amenée (1,5) pour un gaz porteur et un liquide, ainsi qu'une canalisation (9) pour évacuer le mélange gaz/vapeur, caractérisé en ce que le réacteur d'évaporation (8) est constitué par un bloc chauffant (11), qui est pourvu d'une cavité (12) et est disposé dans un boîtier (15) et que dans la cavité (12) est disposé un tube hélicoïdal (13) pour le gaz porteur, qui est relié d'une manière thermoconductrice au bloc chauffant (11), et que sur l'enveloppe extérieure (17) du bloc chauffant (11) est disposé, d'une manière thermoconductrice, un second tube hélicoïdal (14), dans lequel débouchent le système (5) d'amenée du liquide et le tube hélicoïdal (13) pour le gaz de support.
